# FASCICULE DE BREVET EUROPEEN

(11) **EP 0 751 759 B1**
(45) Date de publication et mention de la délivrance du brevet: **15.09.1999**
(21) Numéro de dépôt: 95914382.7
(22) Date de dépôt: 20.03.1995
(51) Int. Cl.: A61K 7/06

(54) **Lotion capillaire a base de minoxidil**
Haarlotion enthaltend Minoxidil
Minoxidil-based capillary lotion

(30) Priorité: 22.03.1994 FR 9403338
(43) Date de publication de la demande: 08.01.1997
(73) Titulaire: Pierre Fabre Dermo-Cosmetique, 92100 Boulogne (FR)
(72) Inventeur: NAVARRO, Roger, F-09100 Pamiers (FR); DELAUNOIS, Marlène, F-31290 Villefranche-de-Lauragais (FR)
(74) Mandataire: Warcoin, Jacques
(86) Numéro de dépôt international: FR9500335
(87) Numéro de publication internationale: WO9525500

(56) Documents cités:
- FR-A- 2 602 421

## Description

L'invention concerne une composition capillaire à base de Minoxidil pour stimuler la croissance des kéranocytes et favoriser la repousse des cheveux.

Il est bien connu aujourd'hui, notamment par le brevet US 4 139 619, que l'utilisation du Minoxidil par voie topique externe permet par traitement du cuir chevelu d'arrêter la chute des cheveux et de permettre leur repousse. De nombreux travaux cliniques ont démontré l'intérêt d'un tel produit et plusieurs spécialités ont été commercialisées à ce jour.

Le Minoxidil ou 6-(1-pipéridinyl)-2,4 pyrimidine diamine 3 oxyde, de formule : 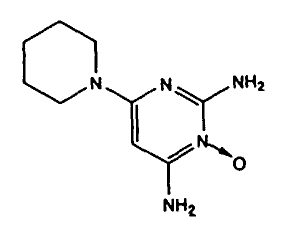 présente un inconvénient majeur en ce qui concerne son utilisation. Sa solubilité dans l'eau étant très faible, il est nécessaire en effet d'utiliser des solvants peu polaires pour dissoudre cette molécule.

Dans le brevet américain précité, on a proposé d'utiliser des solvants du type glycol, comme le propylène glycol ou le polyéthylène glycol ou des solvants du type N-méthyl pyrolidone.

On a aussi décrit des formules utilisant des véhicules du type émulsion où le Minoxidil est soit dissous, soit dispersé sous forme de solution dans les produits plus lipophiles comme des esters de sorbitan ou des alcools gras éthoxylés.

Il existe aussi des onguents associant le Minoxidil à des corps gras comme la lanoline, les huiles de paraffine et la vaseline.

Il est bien clair que ces produits sont très difficiles à utiliser, car leur usage sur le cuir chevelu est très désagréable, le patient refusant d'utiliser des produits aussi gras.

Dans le but d'améliorer ces formulations, on a proposé des formulations associant des solvants de poids moléculaire variant de 200 à 600, comme le propylène glycol ou le polyéthylène glycol, la N-méthyl pyrolidone ou l'éther monoéthylique du diéthylène glycol à l'alcool éthylique ou isopropylique. Ces solvants associés entre eux permettent de solubiliser le Minoxidil et d'assurer sa stabilité dans le temps. Ces formulations contiennent encore cependant des doses importantes de propylène glycol (30 %).

Il est bien connu que malgré la motivation de l'utilisateur, même les lotions associant alcool éthylique et propylène glycol, qui sont considérés comme les produits les moins désagréables, sont abandonnées car les cheveux sont rapidement gras, brillants et d'aspect peu esthétique.

Une solution évidente consisterait à remplacer le polyalcool par un autre alcool, mais la formulation d'une lotion alcool éthylique/eau conduit à l'insolubilité du Minoxidil à 2 % dans un mélange 50/50.

On a également proposé dans le document FR-A-2 602 421 une composition sous forme de gel pour induire et stimuler la croissance des cheveux, comportant un dérivé de pipéridino-pyrimidine, notamment le minoxidil, et un agent gélifiant choisi parmi les hétérobiopolysaccharides et les dérivés de cellulose, notamment les gommes de xanthane engendrées par la bactérie Xanthomonas Campestri.

Par rapport aux lotions classiques qui utilisent des associations alcool/polyalcool, le but de cette invention est la réalisation d'une lotion capillaire agréable d'utilisation, tout en utilisant une quantité beaucoup plus faible de polyalcool, notamment le propylène glycol. La composition capillaire selon l'invention est caractérisée en ce qu'elle consiste en une lotion contenant :
0,1 à 3 % en poids de Minoxidil,
0,1 à 3 % en poids de cyclodextrine,
0,5 à 10 % en poids d'un solvant du Minoxidil,
30 à 50 % en poids d'un alcool,
q.s.p. 100 % d'eau.

Selon un autre aspect de la présente invention, la quantité de cylodextrine présente dans la composition capillaire est telle qu'elle permet de diminuer substantiellement la quantité de solvant du Minoxidil qu'il serait normalement nécessaire d'ajouter pour obtenir une solubilité comparable du Minoxidil en absence de ladite cyclodextrine.

Les cyclodextrines aptes à être utilisées pour la réalisation des compositions capillaires selon l'invention sont bien connues. On pourra en trouver une liste exhaustive dans plusieurs documents qui sont à la disposition de l'homme du métier.

Parmi les cyclodextrines qui sont avantageusement utilisées dans le cadre de la présente invention, on citera les cyclodextrines choisies parmi les α-cyclodextrines, les β-cyclodextrines, les β-cyclodextrines partiellement méthylées et les hydroxypropyl- β-cyclodextrines, seules ou en mélange. Les α-cyclodextrines sont solubles dans l'eau à environ 12,7 %, les β-cyclodextrines sont solubles à environ 1,8 % et les β-cyclodextrines partiellement méthylées solubles à environ 68 %.

On choisira de préférence les cyclodextrines seules ou en mélange dont la solubilité dans l'eau est supérieure à 6 %.

Les β-cyclodextrines partiellement méthylées sont préférées pour les compositions selon l'invention.

Les solvants du Minoxidil sont ceux qui sont généralement connus pour présenter une telle propriété, à savoir les dérivés glycoliques ou la N-méthyl pyrolidone ou les éthers monoéthyliques de diéthylène glycol.

Parmi les dérivés glycoliques, on citera à titre indicatif le propylène glycol, l'éthylène glycol, le dipropylène glycol et en général tous les polyéthylène-glycols de poids moléculaire variant de 200 à 600.

Le propylène glycol est néanmoins le glycol préféré pour la réalisation des compositions selon l'invention.

De préférence, la composition contient de 2 à 8 % de solvant du Minoxidil.

Parmi. les alcools qui sont en fait des monoalcools, on peut citer les alcools en C₂-C₄ et notamment l'alcool éthylique ou l'alcool isopropylique.

Lors d'essais effectués sur des patients en utilisant différentes lotions selon la présente invention, on a trouvé que les compositions capillaires selon l'invention présentaient des qualités particulièrement intéressantes après application, en ce qui concerne les trois aspects suivants :
- aspect non gras,
- temps de séchage de 5 minutes environ,
- facilité de coiffage.

Par ailleurs, 30 minutes après application, la chevelure présentait un aspect naturel, c'est-à-dire que les cheveux étaient "non gras", gonflants, non ternes, doux et faciles à coiffer.

Le lendemain des applications, ce même aspect était parfaitement conservé.

L'invention concerne également un procédé de traitement cosmétique contre l'alopécie consistant à appliquer une dose journalière efficace de Minoxidil sous la forme d'une composition telle que décrite précédemment.

La préparation des compositions selon l'invention s'effectue par mélange des différents ingrédients et plus précisément par dissolution du Minoxidil dans la phase alcoolique puis ajout de ou des cyclodextrines, dudit solvant du Minoxidil et enfin de l'eau.

la lotion obtenue est limpide agréable à utiliser, non grasse et non collante.

Afin d'obtenir un séchage rapide de la composition et la meilleure tolérance possible, la composition doit de préférence avoir un degré alcoolique inférieur à 55 % en volume.

Les exemples ci-après illustrent l'invention sans toutefois la limiter.

### Exemple 1

On réalise par dissolution de Minoxidil dans de l'alcool éthylique à 95 v/v puis ajout des autres ingrédients, une composition comprenant :

| | |
|---|---|
| . Minoxidil | 2 g |
| . β-cyclodextrine (P.M.C.D.) | 1 g |
| . Alcool éthylique à 95 v/v | 42,3 g |
| . Propylène glycol | 5 g |
| . Eau purifiée qsp | 100 ml. |
| P.M.C.D. : cyclodextrine partiellement méthylée. | |

### Exemple 2

On réalise selon le mode opératoire de l'exemple 1 la composition suivante :

| | |
|---|---|
| . Minoxidil | 1 g |
| . β-cyclodextrine (P.M.C.D.) | 1 g |
| . Alcool éthylique à 95 v/v | 40g |
| . Propylène glycol | 10g |
| . Eau purifiée qsp | 100ml. |

### Exemple 3

On réalise selon le mode opératoire de l'exemple 1 la composition suivante :

| | |
|---|---|
| . Minoxidil | 1 g |
| . α-cyclodextrine | 0,5 g |
| . Propylène glycol | 1 g |
| . Alcool éthylique à 95 v/v | 42 g |
| . Eau purifiée qsp | 100ml. |

### Exemple 4

On réalise selon le mode opératoire de l'exemple 1 la composition suivante :

| | |
|---|---|
| . Minoxidil | 2 g |
| . β-cyclodextrine (P.M.C.D.) | 1 g |
| . Propylène glycol | 2 g |
| . Alcool èthylique à 95 v/v | 42,3 g |
| . Eau purifiée qsp | 100ml. |

### Exemple 5

On réalise selon le mode opératoire de l'exemple 1 la composition suivante :

| | |
|---|---|
| . Minoxidil | 2 g |
| . Hydroxypropyl β-cyclodextrine | 1 g |
| . Propylène glycol | 2 g |
| . Alcool éthylique à 95 v/v | 42,3 g |
| . Eau purifiée qsp | 100ml. |

### Exemple 6 (comparatif)

Selon le mode opératoire de l'exemple 1, on réalise la composition suivante :

| | |
|---|---|
| . Minoxidil | 2 g |
| . Alcool éthylique à 95 v/v | 30g |
| . Propylène glycol | 30g |
| . Eau purifiée qsp | 100ml. |

### Exemple 7 - Essai sur le cuir chevelu

Les expériences relatives ci-après ont pour objet d'évaluer comparativement l'acceptabilité cosmétique et l'efficacité des compositions de lotion capillaire des exemples 1 à 6.

L'essai monocentrique a été effectué dans un centre spécialisé sur 12 sujets masculins en double aveugle, randomisé, réalisé en hémi-tête.
. L'essai a duré 8 jours.
. La quantité de composition appliquée par hémi-tête était de 0,5 ml.

Les compositions des exemples 1 à 5 ont un temps de séchage de cinq minutes environ et se sont révélées satisfaisantes en ce qui concerne l'aspect non gras de cheveux et la facilité de coiffage de ceux-ci. Ainsi, 30 minutes après l'application, la chevelure a un aspect naturel, les cheveux sont gonflants, "non gras", non ternes, doux, faciles à coiffer.
. Le lendemain des applications, ces cheveux restent "non gras", "non alourdis", gonflants, doux, brillants, faciles à coiffer, d'aspect naturel.

La composition de l'exemple 1 s'est révélée la plus intéressante par rapport aux trois autres compositions testées :

Les problèmes rencontrés avec la composition de l'exemple 6 sont les suivants :
. Temps de séchage supérieur à 30 minutes,
. Aspect "gras" des cheveux.

Les compositions des exemples selon l'invention se sont révélées, à quantité égale de Minoxidil, aussi efficaces que les mêmes compositions de l'art antérieur.

## Revendications

1. Composition capillaire pour stimuler la croissance des kératinocytes et favoriser la repousse des cheveux, caractérisée en ce qu'elle consiste en une lotion contenant :
0,1 à 3 % en poids de Minoxidil,
0,1 à 3 % en poids de cyclodextrine,
0,5 à 10 % en poids d'un solvant du Minoxidil,
30 à 50 % en poids d'un alcool,
q.s.p. 100 % d'eau.

2. Composition selon la revendication 1, caractérisée en ce qu'elle contient 2 à 8 % en poids d'un solvant du Minoxidil.

3. Composition selon la revendication 1 ou 2, caractérisée en ce qu'elle contient entre 0,5 et 2 % de Minoxidil.

4. Composition selon la revendication 1 à 3, caractérisée en ce que ladite cyclodextrine est choisie parmi les α-cyclodextrines, les β-cyclodextrines, les β-cyclodextrines partiellement méthylées et les hydroxypropyl- β-cyclodextrines, seules ou en mélange.

5. Composition selon la revendication 4, caractérisée en ce que ladite cyclodextrine est choisie parmi les β-cyclodextrines partiellement méthylées.

6. Composition selon l'une des revendications 1 à 5, caractérisée en ce que l'alcool est choisi parmi l'alcool éthylique et l'alcool isopropylique.

7. Composition selon l'une des revendications 1 à 6, caractérisée en ce que le solvant du Minoxidil est choisi parmi les polyéthylène glycols de poids moléculaire variant de 200 à 600, de préférence le propylène glycol.

## Claims

1. Hair composition for stimulating the growth of keratinocytes and for promoting hair regrowth, characterized in that it consists of a lotion comprising:
0.1 to 3% by weight of minoxidil,
0.1 to 3% by weight of cyclodextrin,
0.5 to 10% by weight of a solvent for the minoxidil,
30 to 50% by weight of an alcohol,
q.s. for 100% of water.

2. Composition according to Claim 1, characterized in that it comprises 2 to 8% by weight of a solvent for the minoxidil.

3. Composition according to Claim 1 or 2, characterized in that it comprises between 0.5 and 2% of minoxidil.

4. Composition according to Claims 1 to 3, characterized in that the said cyclodextrin is chosen from α-cyclodextrins, β-cyclodextrins, partially methylated β-cyclodextrins and hydroxypropyl-β-cyclodextrins, alone or as a mixture.

5. Composition according to Claim 4, characterized in that the said cyclodextrin is chosen from partially methylated β-cyclodextrins.

6. Composition according to one of Claims 1 to 5, characterized in that the alcohol is chosen from ethyl alcohol and isopropyl alcohol.

7. Composition according to one of Claims 1 to 6, characterized in that the solvent for the minoxidil is chosen from polyethylene glycols with a molecular weight varying from 200 to 600 and, preferably, propylene glycol.

## Patentansprüche

1. Haarzusammensetzung zur Stimulierung des Keratinocytenwachstums und Förderung des Nachwachsens der Haare, dadurch charakterisiert, daß sie aus einer Lotion besteht, welche enthält:
0,1 bis 3 Gew.-% Minoxidil,
0,1 bis 3 Gew.-% Cyclodextrin,
0,5 bis 10 Gew.-% eines Lösungsmittels für Minoxidil,
30 bis 50 Gew.-% eines Alkohols,
q.s.p. 100% Wasser.

2. Zusammensetzung nach Anspruch 1, dadurch charakterisiert, daß sie 2 bis 8 Gew.-% eines Lösungsmittels für Minoxidil enthält.

3. Zusammensetzung nach Anspruch 1 oder 2, dadurch charakterisiert, dqaß sie zwischen 0,5 und 2 % Minoxidil enthält.

4. Zusammensetung nach Anspruch 1 bis 3, dadurch charakterisiert, daß das Cyclodextrin ausgewält wird aus den α-Cyclodextrinen, den ß-Cyclodextrinen, den partiell methylierten ß-Cyclodextrinen und den Hydroxypropyl-ß-cyclodextrinen, einzeln oder als Gemisch.

5. Zusammensetzung nach Anspruch 4, dadurch charakterisiert, daß das Cyclodextrin ausgewählt wird aus den partiell methylierten Cyclodextrinen.

6. Zusammensetzung nach einem der Ansprüche 1 bis 5, dadurch charakterisiert, daß der Alkohol ausgewählt wird aus Ethylalkohol und Isopropylalkohol.

7. Zusammensetzung nach einem der Ansprüche 1 bis 6, dadurch charakterisiert, daß das Lösungsmittel für Minoxidil ausgewählt wird aus den Polyethylenglykolen mit einem Molekulargewicht von 200 bis 600, bevorzugt Propylenglykol.
